# EUROPEAN PATENT APPLICATION

(11) **EP 1 769 740 A1**
(43) Date of publication of application: **04.04.2007**
(21) Application number: 05256162.8
(22) Date of filing: 03.10.2005
(51) Int. Cl.: A61B 5/0408

(54) **A biomedical electrode, a gel for use in a biomedical electrode and a method of producing a biomedical electrode**

(71) Applicant: Unomedical Limited, Redditch, Worcestershire B98 9N1 (GB)
(72) Inventor: Cummings, Edward Andrew, Armagh Co. Armagh, BT 61 9EP (GB); Southwell, James, Gloucester GL 3 4RH (GB); Wilcox, Alun, Surrey TW10 7LQ (GB); James, Michael, London SW17 0DF (GB); Watkins, Morton, Gloucestershire GL5 4PA (GB)
(74) Representative: Nielsen, Henrik Sten

(57) **Abstract**

A biomedical electrode (10) which comprises a conductive adhesive gel (12) defining opposite top and bottom surfaces. The bottom surface defines a contact area to be contacted with and adhered to a skin surface part of a person. The electrode further comprises a conductive substrate (16) having a conductive face and a non-conductive face and defining a pad portion and a tab portion (18). The pad portion is joint with the top surface and covers a major part thereof and the top surface defines a minor part not covered by the pad portion. A non-conductive pad (20) covers at least the minor part of the top surface of the conductive adhesive gel, and the conductive adhesive gel (12) includes a woven or non-woven web serving to reinforce the conductive adhesive gel. The woven or non-woven web includes hydrophilic fibres such as fibres made from polyester.

## Description

The present invention relates to a biomedical electrode, a gel for use in a biomedical electrode, and a method of producing a biomedical electrode.

Within the technical field of electro-cardiography, biomedical electrodes are used for recording so-called electro-cardiograms. Numerous biomedical electrode designs and structures have been described in numerous patent applications and patents, including EP 0 360 496, EP 0 509 704, US 4,559,996, US 4,539,996, US 4,543,958, US 4.694,835, US 4,852,571, US 1,622,446, US 4,166,465, US 4,852,571, US 4,657,023, US 4,679,563, US 4,922,911, US 5,133,356 and US 5,226,225. Numerous bio-adhesive compositions and biomedical electrodes containing bio-adhesive compositions have further been described in numerous patent applications and patents including WO 00/07638, WO 00/07637, WO 00/07636, WO 00/06215, WO 06214, US 6,792,301, US 6,641,569, US 6,613,030, US 6,592,898, US 6,447,798, EP 1 104 312, EP 1 100 557, EP 1 100 556, EP 1 100 555 and EP 1 100 554.

Reference is made to the above patent applications and the above US patents are hereby incorporated in the present specification by reference.

Through investigation of the prior art of biomedical electrodes, the inventors have deduced certain surprising novel techniques of improving the medical electrodes, firstly, in relation to the mechanical integrity of the biomedical electrode and the ability of the biomedical electrode to prevent an unintentional peeling off of the biomedical electrode, as the biomedical electrode is exposed to a pulling force during use. The biomedical electrodes are conventionally applied to a skin surface part, such as the chest of a patient or person and a clip is fixated to the biomedical electrode or to an outwardly protruding flap of the biomedical electrode. Provided a pull is intentionally applied to the flap, a risk exists that the pull causes the adhesive underlying the connecting flap to peel off from the skin surface of the patient and in doing so, causes the entire biomedical electrode to loose its grip. The reduced contact to the skin surface may turn out to cause inconvenience to the patient and/or clinician, since the measuring apparatus tends to be malfunctioning and not provide an entirely correct measuring, the reduced ability of the prior art biomedical electrodes to withstand pulling forces causing a demounting of the biomedical electrode may make the process of recording an electro-cardiogram time consuming and consequently costly.

An object of the present invention is to provide an improved biomedical electrode as compared to the prior art electrodes and consequently eliminate the above drawbacks of the prior art biomedical electrodes.

A particular advantage of the present invention relates to the fact that a particular reinforcing technique may be integrated into the biomedical electrode without increasing the weight or size of the biomedical electrode to any substantial extent and without influencing the electrical recording capability of the biomedical electrode as compared to the prior art electrode, i.e. consequently without influencing the biomedical electrodes capability of recording voltages and currents generated by the heart of the patient or person wearing the biomedical electrode in electro-cardiography.

A particular feature of the present invention relates to techniques of strengthening the structure of the biomedical electrode by simple applying a double sided adhesive tape or similar high strength adhesive bonding to the biomedical electrode for improving the mechanical properties of the biomedical electrode.

The above objects, the above advantage and the above features together with numerous other objects, advantages and features, which will be evident from the below detailed description of a presently preferred embodiment of the biomedical electrode according to the present invention is in accordance with a first aspect of the present invention obtained by a biomedical electrode comprising:
i) a conductive adhesive gel defining opposite top and bottom surfaces, said bottom surface defining a contact area to be contacted with and adhered to a skin surface part of a person,
ii) a conductive substrate having a conductive face and a non-conductive face and defining a pad portion and a tab portion, said pad portion being joint with said top surface and covering a major part thereof and said top surface defining a minor part not covered by said pad portion,
iii) a non-conductive pad covering at least said minor part of said top surface of said conductive adhesive gel, and
iv) said conductive adhesive gel including a woven or non-woven web serving to reinforce said conductive adhesive gel.

According to the basic teachings of the present invention, it has been realised that the ability of the biomedical electrode to withstand pulling forces applied to the tab portion of the conductive substrate is improved without increasing the size or weight of the biomedical electrode and without influencing the electrical properties of the biomedical electrode by introducing a woven or non-woven web integrally included in the conductive adhesive gel and serving to reinforce the conductive adhesive gel. It is contemplated that a woven or non-woven web serves to distribute any pulling forces applied to the tab portion into the entire conductive adhesive gel rather than to a narrow or limited area below the tab portion, which otherwise would result in a local separation of the conductive adhesive gel from the contact with the skin surface of the patient or person and further on a peeling off of the biomedical electrode.

According to the presently preferred embodiment of the biomedical electrode according to the present invention, the woven or non-woven web includes hydrophilic fibres, such as fibres made from polyester. It is further contemplated that the provision of the woven or non-woven web in the conductive adhesion gel improves the mechanical properties of the biomedical electrode as discussed above by simply reinforcing the conductive adhesive gel by increasing the tear strength of the conductive adhesive gel. Furthermore, since the woven or non-woven web has good tensile strength properties and good tensile strength in both normal direction and also across the width (machine direction and cross-machine direction), hence the biomedical electrode also benefits from the additional strength of the improved tensile strength properties of the conductive adhesive gel, since a polyester, such as a PE or PET web is preferred, the hydrophilic fibres made from PE or PET exhibit good hydrophilic properties providing a high bonding between the hydrophilic fibre web and the hydro gel constituting the conductive adhesive gel. The advantageous bonding between the hydrophilic fibres and the hydro gel provides an overall high integrity between the reinforcing web and the conductive adhesive gel and consequently prevents delamination or separation between the reinforcing web and the conductive adhesive gel. Since the web is advantageously made from hydrophilic fibres, the web tends to be maintained consistently within the embedding conductive adhesive gel without causing the reinforcing web to migrate to the lower surface or the top surface of the conductive adhesive gel. Furthermore, as the hydrophilic fibre web integrally bonds to the hydro gel, the ionic properties of the hydro gel are not affected and the electrical properties, such as the conductance or impedance of the final electrode, are not to any substantial effect influenced by the presence of the reinforcing web.

The advantageous properties of the biomedical electrode implemented in accordance with the teachings of the present invention by the presence of a reinforcing web embedded within the conductive adhesive gel is further supported by the below tests clearly proving a radical increase in the biomedical electrode's ability to stand higher pulling forces as compared to the prior art biomedical electrodes.

According to a further aspect of the present invention, a non-conductive cover pad is provided extending beyond the conductive adhesive gel for providing a freely exposed gripping flip part. Since the presence of the reinforcing web embedded within the conductive adhesive gel improves the biomedical electrode's adhesion to the skin surface part of the patient or person, the biomedical electrode according to the present invention may tend to be fairly difficult to remove from its adhesion to the patient or person without causing unnecessary pain to the patient or person. It is to be understood, as discussed above, that the presence of the reinforcing web improves the peeling off withstanding ability of the biomedical electrode and for allowing the biomedical electrode to be easily peeled off, the freely exposed gripping flip part allows a person such as a nurse to remove the biomedical electrode by peeling off the biomedical electrode by means of the gripping flip part.

According to a particular advantageous aspect of the present invention, it has been realised that adhesion of the biomedical electrode to the supporting surface, i.e. the skin surface of a patient or person by reinforcing the junction between the tab portion and the conductive substrate and/or the junction between the tab portion and the non-conductive part covering the minor part of the top surface of the conductive adhesive gel.

Consequently, according to a second independent aspect of the present invention, a biomedical electrode is provided fulfilling the above object, the above feature and the above advantage together with numerous other objects, advantages and features, which will be evident from the below detailed description of a presently preferred embodiment of the biomedical electrode according to the present invention by a biomedical electrode comprising:
i) a conductive adhesive gel defining opposite top and bottom surfaces, said bottom surface defining a contact area to be contacted with and adhered to a skin surface part of a person,
ii) a conductive substrate having a conductive face and a non-conductive face and defining a pad portion and a tab portion, said pad portion being joint with said top surface and covering a major part thereof and said top surface defining a minor part not covered by said pad portion,
iii) a non-conductive pad covering at least said minor part of said top surface of said conductive adhesive gel, and
iv) said conductive substrate defining a junction between said pad portion and said tab portion, an additional adhesive joint being provided at said junction between said conductive substrate and said conductive adhesive gel, or alternatively and preferably, between said conductive substrate and said cover pad.

The biomedical electrode according to the second aspect of the present invention advantageously also includes the woven or non-woven web characteristic of the biomedical electrode according to the first aspect of the present invention and similarly, the biomedical electrode according to the first aspect of the present invention also advantageously includes the additional adhesive joint characteristic of the biomedical electrode according to a second aspect of the present invention.

The conductive substrate included in the biomedical electrode according to the first and the second aspect of the present invention may advantageously comprise a polyester foil covered by a coated paper providing said non-conductive face and a conductive silver/silver chloride layer and a conductive graphite layer interlayered between said polyester layer and said conductive silver/silver chloride layer.

The present invention also relates to a gel for use in a biomedical electrode or similar device, which gel provides the above described advantages and fulfils the above object, feature, as a gel being a water based, conductive, adhesive gel including a woven or non-woven web of hydrophilic fibres such as PE fibres.

Examples of possible conductive adhesive gels and hydrophilic webs are described below.

Possible web materials are polymers, such as polyester, crosslinked poly(N-vinyl lactam), crosslinked polyacrylamide; crosslinked polyacrylic acid; crosslinked poly(2-acrylamide-2-methylpropanesulfonic acid), crosslinked copolymers of the acid, or mixtures thereof. Of these polymers polyester is especially preferred.

The nonwoven hydrophilic web is electrically inactive and reinforces the hydrogel so as to provide superior strength and cohesion. The hydrophilic web made of nonwoven polyester, prefably in combination with polyethylene, provides the hydrogel with increased adhesive and tensile strength relative to prior art hydrogels.

Non-limiting examples of polymeric materials suitable for use in the hydrophilic web include polyolefins (e.g., polyethylene and polypropylene), polyesters, and polyurethanes, polyethylene terephthalate, block copolymers comprising aliphatic polyethers and aromatic polyesters, block copolymers from aliphatic polyether-s and polyamides, polyamides, polyimides, polyurethanes, or hydrocarbon polymers such as polyethylene and polypropylene, synthetic hydrocarbon elastomers, as well as natural rubber.

The hydrogel of the present invention may be based on prior art substances commonly known to form hydrogels when contacted with water, e.g. polysaccharide hydrogels. Polysaccharide hydrogels include, by way of example, polysaccharides such as alginic acids and their alkali metal salts, said alginic acids consisting of various copolymer segments of D-mannuronic acid and L-glucuronic acid, depending upon their natural origin. Other naturally occurring water-soluble seaweed gums suitable for this use are the carrageenan types, which comprise linear polysaccharides of alternating 1,3-linked β-D-galactopyranosyl and 1,4-linked α-D-galactopyranosyl units. Due to their half-ester sulfate groups the carrageenans are anionic polyelectrolytes, and their sodium salts form water-soluble stable hydrogels which can be readily incorporated as the hydrogel combinations depicted by the present invention. Other suitable polysaccharide hydrogels comprise hyaluronic acids and their alkali metal salts, chondroitin sulfates and the corresponding alkali metal sulfates and carboxylates represent further useful water-soluble hydrogel polymers as components of the present invention. Typical water-soluble derivatives from celluloses that are suitable as hydrogel polymers of the instant invention comprise water-soluble sodium carboxymethyl celluloses, as well as water-soluble hydroxyethyl celluloses or hydroxypropyl celluloses, and the like, all well known in the art. Most of these materials exhibit a very low order of toxicity and are usually biocompatible. Further water-soluble polymers useful as hydrogel polymer ingredients of the present invention are poly(oxyethylene) homopolymers having molecular weights of from about 100,000 or lower, to about 5,000,000 or higher, which are known as POLYOX® polymers (UNION CARBIDE). Additional typical water-soluble polymers useful as hydrogel polymers of the present invention include poly(N-vinylpyrrolidones) having molecular weights of from about 10,000 to about 340,000 (such as the PVP polymers available from GAF Corp.). In particular, the conductive adhesive gel may advantageously be one of the conductive adhesive gels described in the above-mentioned patent applications and patents relating to bio-adhesive compositions and biomedical electrodes containing bio-adhesive compositions.

The present invention further relates to methods of producing biomedical electrodes.

The above objects, the above advantages and the above features together with numerous other objects, advantages and features, which will be evident from the below detailed description of a presently preferred embodiment of the biomedical electrode according to the present invention is in accordance with a third aspect of the present invention obtained by a method of producing a biomedical electrode comprising:
i) providing a conductive adhesive gel defining opposite top and bottom surfaces, said bottom surface defining a contact area to be contacted with and adhered to a skin surface part of a person,
ii) providing a conductive substrate having a conductive face and a non-conductive face and defining a pad portion and a tab portion,
iii) joining said pad portion with said top surface so as to cover a major part thereof and to provide a minor part thereof not covered by said pad portion,
iv) providing a non-conductive pad and joining said non-conductive pad with at least said minor part of said top surface of said conductive adhesive gel, and
v) providing a woven or non-woven web and embedding said woven or non-woven web in said conductive adhesive gel serving to reinforce said conductive adhesive gel.

The above objects, the above advantages and the above features together with numerous other objects, advantages and features, which will be evident from the below detailed description of a presently preferred embodiment of the biomedical electrode according to the present invention is in accordance with a fourth aspect of the present invention obtained by a method of providing a biomedical electrode comprising:
i) providing a conductive adhesive gel defining opposite top and bottom surfaces, said bottom surface defining a contact area to be contacted with and adhered to a skin surface part of a person,
ii) providing a conductive substrate having a conductive face and a non-conductive face and defining a pad portion and a tab portion,
iii) joining said pad portion with said top surface so as to cover a major part thereof and so as to provide a minor part thereof not covered by said pad portion,
iv) providing a non-conductive pad and joining said non-conductive pad with at least said minor part of said top surface of said conductive adhesive gel, and
v) providing an additional adhesive joint and applying said additional adhesive joint at a junction between said pad portion and said tab portion so as to join at said junction said conductive substrate with said conductive adhesive gel, or alternatively and preferably, so as to join said conductive substrate with said cover pad.

The present invention is now to be further described with reference to the drawings, in which
Fig. 1 is a perspective top view of a first and presently preferred embodiment of a biomedical electrode according to the present invention,
Fig. 2 is a bottom perspective view of the first and presently preferred embodiment of the biomedical electrode according to the present invention,
Fig. 3 is an exploded view of the first and presently preferred embodiment of the biomedical electrode according to the present invention also shown in Figs. 1 and 2,
Fig. 4 is a vertical sectional view of the first and presently preferred embodiment of the biomedical electrode according to the present invention also shown in Figs. 1-3,
Fig. 5 is a perspective view illustrating a set of biomedical electrodes according to the present invention provided on a common supporting release cover foil,
Fig. 6 is a diagrammatic view illustrating a set of tests performed with different embodiments of the biomedical electrode according to the present invention, and
Fig. 7 is a schematic, perspective and diagrammatic view illustrating a plant for the manufacturing of the biomedical electrode according to the present Invention.

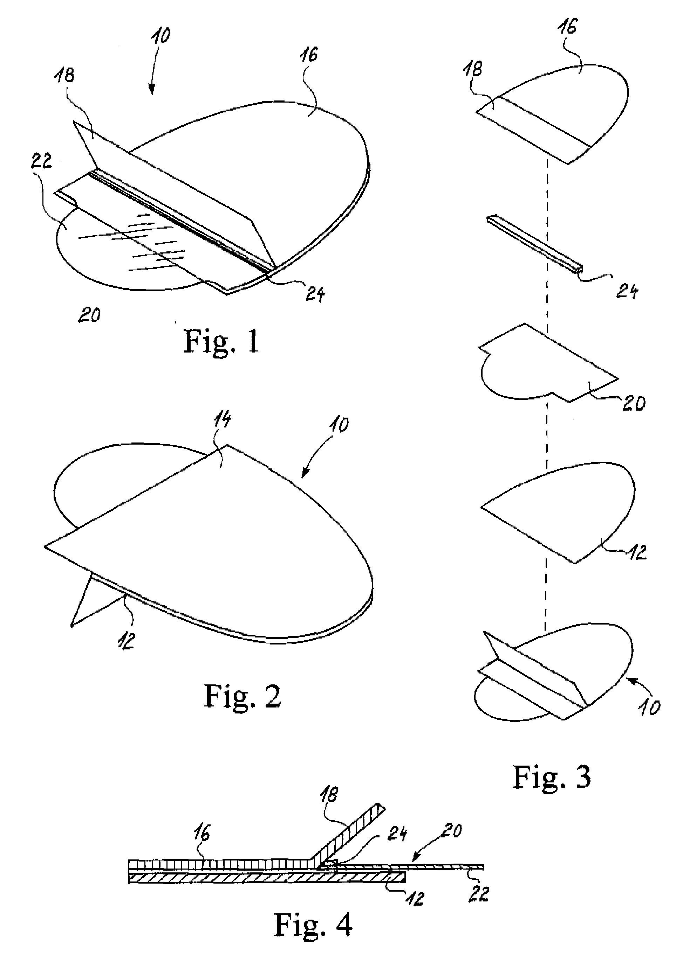

In Fig. 1, 2, 3 and 4, a first and presently preferred embodiment of a biomedical electrode according to the present invention is shown designated the reference numeral 10 in its entirety. In Fig 1, the biomedical electrode 10 is shown as viewed from above, and in Fig. 2, the biomedical electrode is shown as viewed from below. The terms top and bottom, upper, lower, internally, externally etc. refer, unless the context defines a different meaning of the terms, to the intentional application of the biomedical electrode, in which application the biomedical electrode is adhered to a skin surface part of a person or patient, as the bottom or lower surface of the electrode faces and adheres to the skin surface part of the person or patient or faces inwardly towards the skin surface part of the person or patient, whereas the top or upper surface of the biomedical electrode faces upwardly or externally relative to the skin surface part of the person or patient.

The biomedical electrode 10 basically comprises a conductive adhesive gel such as water based and/or skin friendly hydro colloid, in which a woven or non-woven web of hydrophilic fibres such as polyester fibres are embedded for improving the mechanical strength and the integrity of the conductive adhesive gel. The bottom surface of the conductive adhesive gel 12 shown in Fig. 2 is exposed and designated the reference numeral 14. The conductive adhesive gel 12 generally has the configuration of one half of an ellipse, which one half is produced by cutting the ellipse perpendicular to the major axis of the ellipse. On the top surface of the conductive adhesive gel 12, a major part is provided at the rounded end of the conductive adhesive gel, on which major part a conductive substrate 16 is applied and adhered providing a freely extending flap 18, which is separated from the conductive adhesive gel by a non-conductive cover pad 20.

At the transition from the non-conductive cover pad 20 to the flap 18 and the remaining major part of the conductive substrate 16, a high strength adhesive connection 24 is provided serving to increase the fixation of the conductive substrate 16 to the conductive adhesive gel 12 in the intentional application of the biomedical electrode.

The non-conductive cover pad 20 provides an outwardly extending and freely exposed gripping flip part 22 by means of which the entire medical electrode may be removed from its initial release cover foil and also from its application to a skin surface part of a patient or person.

In Fig. 3, the various components of the biomedical electrode shown in Figs. 1 and 2, are illustrated in an exploded view and in Fig. 4, the biomedical electrode 10 is shown in a vertical sectional view.

In the intentional application of the biomedical electrode according to the present invention, the biomedical electrode 10 is applied to a skin surface part of a patient or person as the lower exposed surface 14 of conductive adhesive gel 12 is brought into contact with the skin surface part of a patient or person and caused to adhere to the skin surface part. A contacting element such as a clip having an electrical wire integrally connected to the clip for establishing electrically conductive connection to an external measuring equipment is fixated to the flap 18, which flap has its lower surface exposed for conducting current to or from the conductive adhesive gel through an integral electrically conductive layer of the conductive substrate 16. As the clip is fixated to the flap 18, a risk of tearing off the biomedical electrode potentially exists, and a risk of tearing off the conductive substrate 16 from the conductive adhesive gel also exists.

For improving the mechanical strength of the bonding between the conductive substrate and the conductive adhesive gel, the adhesive connection 24 is provided, which band prevents the conductive substrate 16 from being peeled off the supporting conductive adhesive gel 12.

In addition, the presence of the woven or non-woven integral web of hydrophilic fibres included in the conductive adhesive gel reinforces the gel and contributes to a distribution of any pulling forces introduced into the flap 18 from causing the conductive adhesive to loosen its grip below the point or line of attack or forces in the conductive adhesive gel.

Due to the improved adhesion and stable fixation of the biomedical electrode to the skin surface part of a patient or person, the non-conductive cover pad is provided with the outwardly extending and freely exposed gripping flip part 22 for allowing a person such as a nurse to easily remove the biomedical electrode from its fixation to the skin surface part of a patient or person by gripping the exposed gripping flip part 22 and peeling off the biomedical electrode from the linear boundary line of the conductive adhesive gel 12. In this context, the adhesion between the components of the biomedical electrode relative to the adhesion between the conductive adhesive gel to the skin surface part of the patient or person is of course of the outmost importance, as the adhesion between the non-conductive cover pad 20 and the conductive adhesive gel 12 has to be larger than the force needed to peel off the conductive adhesive gel 12 from the skin surface part of the patient or person and similarly, the connection between the conductive substrate 16 and the conductive adhesive gel 20 established by the presence of the high strength adhesive connection 24 also has to be larger than the force needed to peel off the conductive adhesive gel 12 from the skin surface part of the patient or person for preventing the entire biomedical electrode from disintegrating during use.

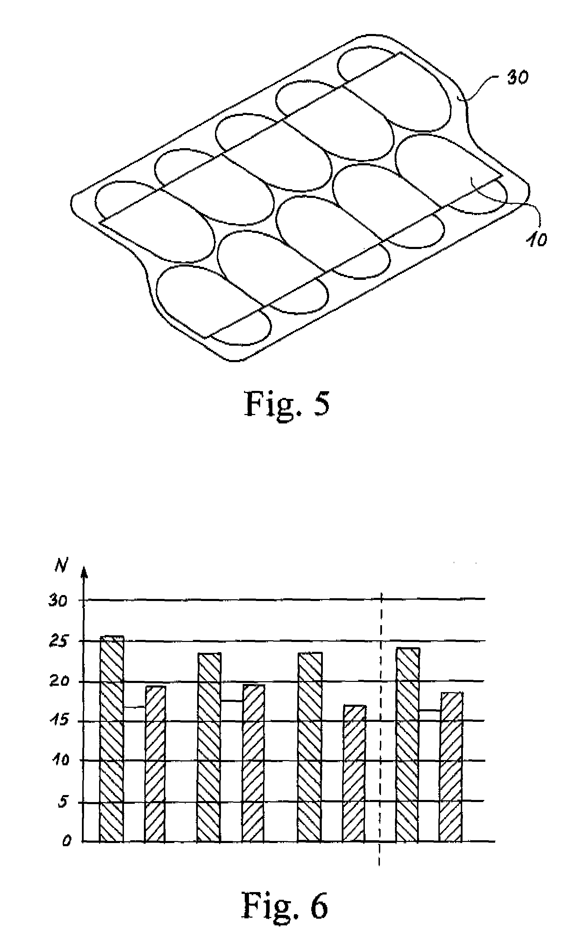

In Fig. 5, a total of 10 biomedical electrodes 10 according to the present invention is shown supported on a common release cover foil 30.

In Fig. 6, a diagram is shown illustrating the results of three tests demonstrating the improvement of the peeling off capability of the biomedical electrode by the provision of the reinforcing hydrophilic fibre mesh or web included in the conductive adhesive gel. All pulling tests described in the present application were performed in accordance with the standard FTM2, FINAT test method No. 2 peel adhesion (90°) at 300mm/min. described in FINAT Technical Handbook, 5th Edition, 1999, page 8-10 (Exhibit 1). A total of three tests were conducted, each relating to three different test variants. In the first variant shown in the left hand column in each of the three test readouts, the hydrophilic or polyester web is included in the conductive adhesive gel in combination with the reinforcing double sided adhesive tape 24 improving the fixation of the conductive substrate 16 at the line of force impact from the web 18 into the conductive adhesive gel 12. In the second test variant, the hydrophilic fibre mesh is omitted and in the third test variant, a different double sided tape is used. The fourth set of columns illustrates the average of the three tests.

From the three tests, it is clearly deduced that the presence of the double sided adhesive tape improves the ability of the biomedical electrode to withstand the peeling off force induced into the web 18 by pulling since the use of different double sided tapes clearly influences the peeling off or separation force necessitating to cause a separation of the biomedical electrode 10 from the supporting surface by the conductive adhesive gel loosing its grip from the supporting surface. It is to be understood that the tests were all conducted without any disintegration of the test variants of biomedical electrodes as the maximum forces illustrated in Fig. 6 represents the forces causing the biomedical electrode in its entirety to loose its grip to its supporting surface.

The left hand columns of the three tests and also of the average of the three tests clearly demonstrate that the presence of the reinforcing hydrophilic fibre mesh or web embedded within the conductive adhesive gel improves the ability of the biomedical electrode to adhere to the supporting surface, which is contemplated to be caused by a distribution effect or reinforcing effect as the mesh simply transforms the unilateral or vertical force generated by pulling the tab 18 to a wider area of contact to the supporting surface and in doing so, prevents the biomedical electrode to loose its grip by a central peeling off of the biomedical electrode below the improvement of the peeling off capability of the biomedical electrode according to the present invention by the provision of the double sided adhesive tape 24 is further supported by the test results listed in table 1 below. In the table, the first line refers to a set of tests, in which the double sided adhesive tape was included in the biomedical electrode, in which case the average force for causing the biomedical electrode to loose its grip from its supporting surface when pulled in the tab 18 was 22.2 N, whereas provided the second line represents the results when the double sided adhesive tape was omitted. The force produced by pulling in the tab 18 for causing the biomedical electrode to disintegrate was 9.25 N, as the conductive adhesive gel 20 was separated from the conductive substrate 16 and consequently left on the supporting surface. Consequently, the omission of the double sided adhesive tape reduces the bonding force between the conductive substrate and the conductive adhesive gel 20 to a force lower than the adhesion between the conductive adhesive gel 20 and the supporting surface. In the third line, the peeling off force by lifting the biomedical electrode off the supporting surface by gripping the gripping flip part 22 was recorded and averaged to 6.38 N.

**TABLE 1: FORCE TO REMOVE GEL AND DOUBLE SIDED ADHESIVE TAPE**

| | 1 | 2 | 3 | 4 | 5 | 6 | AVE |
|---|---|---|---|---|---|---|---|
| Force to remove biomedical electrode including double sided adhesive tape | 22.3 | 22.2 | 24.6 | 22.3 | 22 | 22.9 | 22.72 |
| Force to remove biomedical electrode without double sided tape | 9.34 | 10.3 | 8.57 | 9.02 | 9.29 | 8.95 | 9.25 |
| Force to peel off biomedical electrode by means of gripping flip part 22 | 6.46 | 7.39 | 6.17 | 6.21 | 6.05 | 5.97 | 6.38 |

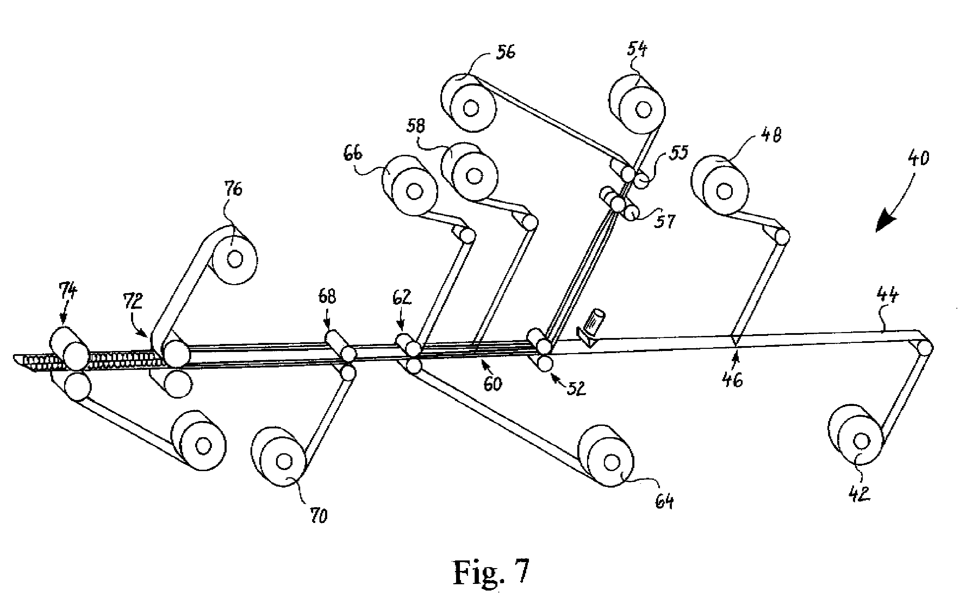

In Fig. 7, a schematic diagram illustrates a plant of manufacturing the biomedical electrode 10 according to the present invention. In Fig. 7, the line of production is operated from right to left. The plant is, in its entirety designated the reference numeral 40 and comprises an unwind station 42 from which the conductive adhesive gel is unwound as the conductive adhesive gel is sandwiched between a top plastics liner and a bottom paper liner.

The sandwich of the bottom paper liner, the conductive adhesive gel and top plastics liner is designated the reference numeral 42 and is introduced into a peeling off station indicated by 46, in which the top plastics liner is removed and wound on to a winding station 48.

While still supported on the bottom paper liner, the conductive adhesive gel integrally including the reinforcing hydrophilic fibre web and designated the reference numeral 50 is introduced into a station 52, in which a polyester foil is supplied from a polyester unwind station 54, which polyester foil eventually provides the non-conductive pad 20.

In addition to the polyester foil, two lines of double sided adhesive tape are supplied from a double-sided adhesive tape unwind station 56 and applied to the top surface of the polyester foil by means of a pair of rollers 55 from which the polyester foil is introduced into a pair of cutting rollers 57, which cut centrally the polyester foil for separating the polyester foil having the double sided adhesive tape applied to the top surface thereof into two separate bands to be positioned edgewise on the top surface of the conductive adhesive gel 50.

By means of a further winding station 58, two release liners or release papers of the double sided adhesive tape supplied from the station 56 are removed in a station designated 60. In a further station 62, the supporting bottom paper of the conductive adhesive gel is removed and wound on to a further winding station 64 and at the same time or shortly before the removal of the bottom supporting paper, the conductive substrate is supplied from an unwind station 66 and applied on top of the two uncovered double sided adhesive tapes and also on top of the exposed conductive gel 50.

In a further station 68, a product release liner is contacted with the bottom surface of the conductive adhesive gel as the product release foil is supplied from an unwind station 70.

Finally, the production plant includes two rotary cutter stations 72 and 74, which may possibly be integrated into a single composite rotary cutter station.

In the first rotary cutter station 72, the conductive substrate, the conductive adhesive gel and the non-conductive polyester foil are cut through and the waste material is wound on to a winding station 76. In the second rotary cutter station 74, the product release liner is cut through for cutting the finalised product into the set of 10 biomedical electrodes illustrated in Fig. 5.

The above-described first and presently preferred embodiment of the biomedical electrode according to the present invention was made from the following components which were applied in the below quantities as coatings.

### Example

The conductive adhesive gel was hydro gel of the following composition and including a polyester web having a surface weight of 26 g/m² and was coated in a coating layer of a coating weight 0.9 kg/m²-1.0 kg/m² (amount of conductive adhesive gel including polyester web). The conductive substrate was composed of a total of six layers produced in separate coating processes. The bottom layer facing and contacting the conductive adhesive gel was a conductive silver/silver chloride (AG/AGCI) layer applied in an amount of 2 g/m². On top of the conductive silver/silver chloride layer, a conductive graphite layer was applied in an amount of 10 g/m². Further on top of the conducting graphite layer, four non-conductive layers were applied, firstly a polyester layer in an amount of 17 g/m², secondly, a water based adhesive in an amount of 3.5 g/m², thirdly, a coated paper layer in an amount of 60 g/m² and finally, printed graphics in an amount of 12 g/m².

The polyester web 20 was a 50 µm non-siliconised polyester web of a width of 40 mm.

The release liner was a 97 mm wide, 125 µm thick siliconised polyester foil or polyethylene 20gsm/bleach kraft paper 120gsm/polyethylene 20gsm.

The double adhesive tape 24 was an acrylic adhesive tape 30 gsm.

The polyester web was supplied from the company HDK Industries, Inc. and the product code 5722 and including PE/PET fibre. The web had the following properties:
*Basic weight*
   26 g
*Thickness:*
   0.14 mm
*Tensile strength:*
   Machine direction: 61 N/50 mm, Elongation: 37%
   Cross Direction: 24 N/50 mm, Elongation: 49%

Although the present invention has above been described with reference to a specific and presently preferred embodiment of the biomedical electrode according to the present invention, it is to be understood that the present invention may be implemented in numerous other embodiments and variants, which would be obvious to a person having ordinary skill in the art. Such variants and modifications are to be construed part of the present invention, as defined in the appending patent claims.

## Claims

1. A biomedical electrode comprising:
i) a conductive adhesive gel defining opposite top and bottom surfaces, said bottom surface defining a contact area to be contacted with and adhered to a skin surface part of a person,
ii) a conductive substrate having a conductive face and a non-conductive face and defining a pad portion and a tab portion, said pad portion being joint with said top surface and covering a major part thereof and said top surface defining a minor part not covered by said pad portion,
iii) a non-conductive pad covering at least said minor part of said top surface of said conductive adhesive gel, and
iv) said conductive adhesive gel including a woven or non-woven web serving to reinforce said conductive adhesive gel.

2. The biomedical electrode according to claim1, said woven or non-woven web include hydrophilic fibres such as fibres made from polyester.

3. The biomedical electrode according to claim 1 or 2, said non-conductive cover pad extending beyond said conductive adhesive gel for providing a freely exposed gripping flip part.

4. A biomedical electrode comprising:
i) a conductive adhesive gel defining opposite top and bottom surfaces, said bottom surface defining a contact area to be contacted with and adhered to a skin surface part of a person,
ii) a conductive substrate having a conductive face and a non-conductive face and defining a pad portion and a tab portion, said pad portion being joint with said top surface and covering a major part thereof and said top surface defining a minor part not covered by said pad portion,
iii) a non-conductive pad covering at least said minor part of said top surface of said conductive adhesive gel, and
iv) said conductive substrate defining a junction between said pad portion and said tab portion, an additional adhesive joint being provided at said junction between said conductive substrate and said conductive adhesive gel, or alternatively and preferably, between said conductive substrate and said cover pad.

5. The biomedical electrode according to claim 4, said conductive adhesive gel including a woven or non-woven web serving to reinforce said conductive adhesive gel.

6. The biomedical electrode according to any of the claims 4 or 5, said woven or non-woven web include hydrophilic fibres such as fibres made from polyester.

7. The biomedical electrode according to any of the claims 4-6, said non-conductive cover pad extending beyond said conductive adhesive gel for providing a freely exposed gripping flip part.

8. The biomedical electrode according to any of the claims 1-3, said conductive substrate defining a junction between said pad portion and said tab portion, an additional adhesive joint being provided at said junction between said conductive substrate and said conductive adhesive gel, or alternatively and preferably, between said conductive substrate and said cover pad.

9. The biomedical electrode according to any of the claims 1-8, said conductive substrate comprising a polyester foil covered by a coated paper providing said non-conductive face and a conductive silver/silver chloride layer and a conductive graphite layer interlayered between said polyester layer and said conductive silver/silver chloride layer.

10. A gel for use in a biomedical electrode or similar device, said gel being a water based, conductive adhesive gel including a woven or non-woven web of hydrophilic fibres such as polyester fibres.

11. The gel according to claim 10, further comprising any of the features of the conductive adhesive gel of the biomedical electrode according to any of the claims 1-9.

12. A method of producing a biomedical electrode comprising:
i) providing a conductive adhesive gel defining opposite top and bottom surfaces, said bottom surface defining a contact area to be contacted with and adhered to a skin surface part of a person,
ii) providing a conductive substrate having a conductive face and a non-conductive face and defining a pad portion and a tab portion,
iii) joining said pad portion with said top surface so as to cover a major part thereof and to provide a minor part thereof not covered by said pad portion,
iv) providing a non-conductive pad and joining said non-conductive pad with at least said minor part of said top surface of said conductive adhesive gel, and
v) providing a woven or non-woven web and embedding said woven or non-woven web in said conductive adhesive gel serving to reinforce said conductive adhesive gel.

13. The method according to claim12, said woven or non-woven web include hydrophilic fibres such as fibres made from polyester.

14. The method according to claim 12 or 13, said non-conductive cover pad being provided extending beyond said conductive adhesive gel for providing a freely exposed gripping flip part.

15. A method of providing a biomedical electrode comprising:
i) providing a conductive adhesive gel defining opposite top and bottom surfaces, said bottom surface defining a contact area to be contacted with and adhered to a skin surface part of a person,
ii) providing a conductive substrate having a conductive face and a non-conductive face and defining a pad portion and a tab portion,
iii) joining said pad portion with said top surface so as to cover a major part thereof and so as to provide a minor part thereof not covered by said pad portion,
iv) providing a non-conductive pad and joining said non-conductive pad with at least said minor part of said top surface of said conductive adhesive gel, and
v) providing an additional adhesive joint and applying said additional adhesive joint at a junction between said pad portion and said tab portion so as to join at said junction said conductive substrate with said conductive adhesive gel, or alternatively and preferably, so as to join said conductive substrate with said cover pad.

16. The method according to claim 15, said conductive adhesive gel including a woven or non-woven web serving to reinforce said conductive adhesive gel.

17. The method according to any of the claims 15 or 16, said woven or non-woven web include hydrophilic fibres such as fibres made from polyester.

18. The biomedical electrode according to any of the claims 15-17, said non-conductive cover pad being provided extending beyond said conductive adhesive gel for providing a freely exposed gripping flip part.

19. The method according to any of the claims 12-14, further comprising providing an additional adhesive joint and applying said additional adhesive joint at a junction between said pad portion and said tab portion so as to join at said junction said conductive substrate and said conductive adhesive gel, or alternatively and preferably, between said conductive substrate and said cover pad.
